# FASCICULE DE BREVET EUROPEEN

(11) **EP 2 506 907 B1**
(45) Date de publication et mention de la délivrance du brevet: **02.10.2013**
(21) Numéro de dépôt: 10805614.4
(22) Date de dépôt: 29.11.2010
(51) Int. Cl.: A61M 16/00, F04D 25/08, F04D 29/66

(54) **APPAREIL DE DÉLIVRANCE RÉGULÉE D'UN GAZ, NOTAMMENT APPAREIL D'ASSISTANCE RESPIRATOIRE**
VORRICHTUNG FÜR GEREGELTE GASABGABE, IM BESONDEREN FÜR EINE VORRICHTUNG FÜR UNTERSTÜTZTES ATMEN
APPARATUS FOR THE REGULATED SUPPLY OF A GAS, IN PARTICULAR AN ASSISTED BREATHING APPARATUS

(30) Priorité: 02.12.2009 FR 0905819
(43) Date de publication de la demande: 10.10.2012
(73) Titulaire: Air Liquide Medical Systems S.A., 92182 Antony Cedex (FR)
(72) Inventeur: GRASMUCK, Gilbert, F-31880 La Salvetat Saint Gilles (FR)
(74) Mandataire: Pittis, Olivier
(86) Numéro de dépôt international: PCT/FR2010/052551
(87) Numéro de publication internationale: WO 2011/067522

(56) Documents cités:
- WO-A1-99/22793
- WO-A1-2008/131202
- WO-A2-2007/024955
- FR-A1- 2 843 305
- FR-A1- 2 908 482
- FR-A1- 2 910 081
- US-B1- 6 837 260

## Description

La présente invention concerne un appareil de délivrance régulée d'un gaz, notamment un appareil d'assistance respiratoire.

Il est connu d'utiliser un appareil de délivrance régulée d'un gaz, notamment un appareil d'assistance respiratoire, comprenant un ventilateur constitué essentiellement par un moteur, une roue à ailettes et une volute contenant cette roue, un conduit de refroidissement du moteur du ventilateur, et un boîtier d'insonorisation contenant le ventilateur et le conduit de refroidissement du moteur, comme par exemple décrit par le document FR-A-2908482.

Le type de moteur de ventilateur utilisé et la rotation rapide de ce moteur induisent un échauffement important du moteur, dont le refroidissement est un point critique. En effet, le bon refroidissement de ce moteur a une incidence directe sur les performances et la longévité de l'appareil.

Or, les appareils existants ne donnent pas parfaitement satisfaction de ce point de vue.

De plus, les appareils existants ont des structures relativement complexes impliquant un nombre de pièces élevé, des difficultés d'assemblage et une durée d'assemblage importante.

En outre, la conception, sur ce type d'appareil, d'une entrée d'air permettant à la fois l'obtention d'un faible niveau sonore et d'une perte de charge minimisée est difficile.

La présente invention vise à remédier à l'ensemble de ces inconvénients, c'est-à-dire en particulier de proposer un appareil de délivrance d'un gaz, tel un appareil d'assistance respiratoire, dans lequel un refroidissement suffisant et efficace du moteur est assuré, et ayant par ailleurs une architecture simple qui ne complique pas son assemblage.

La solution selon la présente invention est un appareil de délivrance, de préférence régulée, d'un gaz, en particulier un appareil d'assistance respiratoire, comprenant un ventilateur comprenant un moteur, une roue à ailettes et une volute contenant cette roue, un conduit de refroidissement du moteur, et un boîtier d'insonorisation contenant le ventilateur et le conduit de refroidissement du moteur,le conduit de refroidissement du moteur présentant une forme ajustée à la forme du moteur et de la volute du ventilateur, de sorte que les parois délimitant ce conduit de refroidissement du moteur s'étendent à proximité de la paroi du moteur et de la volute, ménageant ainsi un conduit d'écoulement autour, de préférence tout autour, de ce moteur et de cette volute, caractérisé en ce que l'appareil comprend deux parties de boîtier, dont l'une inclut un ensemble comprenant une paroi de fond et au moins une paroi de côté, formant une partie du boîtier d'insonorisation et un ensemble de parois formant une partie du conduit de refroidissement du moteur, et dont l'autre inclut un ensemble comprenant une paroi de fond et au moins une paroi de côté, formant la partie complémentaire du boîtier d'insonorisation, et un ensemble de parois formant la partie complémentaire du conduit de refroidissement du moteur, lesdites parties du boîtier d'insonorisation et du conduit de refroidissement du moteur d'une même partie de boîtier formant corps l'une avec l'autre, les deux parties de boîtier étant assemblables l'une à l'autre autour du ventilateur de manière à former conjointement ledit conduit de refroidissement du moteur et ledit boîtier d'insonorisation.

L'appareil selon l'invention comprend ainsi un conduit de refroidissement du moteur obligeant le fluide, c'est-à-dire le gaz, entraîné par la roue à ailettes à s'écouler à proximité immédiate des parois externes du moteur et de la volute, et donc assurant un refroidissement efficace de ces dernières, tout en ayant ce conduit de refroidissement du moteur formé en une seule et même pièce avec le boîtier d'insonorisation, permettant ainsi, par convection forcée, l'évacuation de la chaleur générée par le moteur. Un refroidissement optimal du moteur de ventilateur est ainsi obtenu.

L'assemblage de l'appareil est rendu possible par la conception du conduit de refroidissement du moteur et du boîtier d'insonorisation sous forme des deux parties de boîtier précitées. Cette conception, outre la performance obtenue en terme de refroidissement du moteur, a également pour avantages que le nombre de pièces constituant l'appareil est notablement réduit, qu'il est obtenu une plus grande facilité d'assemblage, et donc une durée d'assemblage réduite, et qu'une quantité moindre de matière est nécessaire pour constituer le conduit de refroidissement du moteur et le boîtier d'insonorisation.

En outre, les parties de boîtier peuvent être aménagées par moulage, ce qui permet une possibilité augmentée de positionnement du conduit de refroidissement du moteur dans le boîtier d'insonorisation et une possibilité d'intégrer à l'appareil de façon optimale une entrée de fluide à forte atténuation acoustique et à perte de charge minimisée.

De préférence, les parties de boîtier sont des demi-boîtiers assemblables l'un à l'autre selon un plan d'assemblage passant sensiblement par l'axe longitudinal du conduit de refroidissement du moteur, c'est-à-dire l'axe avec lequel l'axe de rotation de la roue à ailettes du ventilateur est confondu lorsque le ventilateur est en position de montage dans ledit conduit de refroidissement du moteur.

Selon une autre possibilité, les parties de boîtier sont assemblables l'une à l'autre selon un plan d'assemblage sensiblement perpendiculaire à l'axe longitudinal du conduit de refroidissement du moteur, c'est-à-dire l'axe avec lequel l'axe de rotation de la roue à ailettes du ventilateur est confondu lorsque le ventilateur est en position de montage dans ledit conduit de refroidissement du moteur.

La mise en place du ventilateur dans un premier demi-boîtier est ainsi facilitée, ainsi que l'assemblage du deuxième demi-boîtier au premier demi-boîtier.

De préférence, chaque partie de boîtier forme au moins une partie de logement, et les deux parties de logement forment conjointement, après assemblage des parties de boîtier, un logement propre à recevoir de manière ajustée une partie correspondante du ventilateur, soit directement, soit avec interposition d'un élément de montage, notamment un élément souple d'amortissement de vibrations.

Ainsi, non seulement le montage du ventilateur dans le conduit de refroidissement de moteur est facilité, mais également et surtout, il est obtenu un parfait amortissement des vibrations générées par le ventilateur.

Chaque partie de boîtier peut notamment comprendre une partie de logement inférieur, et les deux parties de logement inférieur forment conjointement, après assemblage des parties de boîtier, un logement apte à recevoir de manière ajustée l'extrémité du moteur opposée à la volute ou une pièce fixée à cette extrémité.

Une parfaite immobilisation de cette extrémité du moteur est ainsi obtenue, de manière simple. Ledit logement peut notamment être aménagé dans la paroi latérale du boîtier voisine de l'extrémité inférieure du conduit de refroidissement du moteur.

Chaque partie de boîtier peut également comprendre une partie de logement de tubulure de sortie de volute, les deux parties de logement de tubulure de sortie de volute formant conjointement, après assemblage des parties de boîtier, un logement apte à recevoir de manière ajustée la tubulure de sortie de la volute, avec interposition d'une garniture de montage et d'amortissement.

Chaque partie de boîtier peut aussi comprendre une partie de logement de calage de ventilateur, les deux parties de logement de calage de ventilateur formant conjointement, après assemblage des parties de boîtier, un logement apte à recevoir de manière ajustée un plot de calage de ventilateur solidaire de la volute, avec interposition d'une garniture de montage et d'amortissement.

Chaque partie de logement de calage de ventilateur peut notamment être aménagée en un emplacement du conduit de refroidissement du moteur diamétralement opposé à la partie de logement de tubulure de sortie de volute.

L'assemblage des deux parties de boîtier peut être réalisé par tout moyen, notamment par des vis ou par collage.

Selon le cas, l'appareil de l'invention peut aussi comprendre l'une ou plusieurs des caractéristiques suivantes :
- il comprend deux parties de boîtier, dont l'une inclut un ensemble constitué d'une paroi de fond et de plusieurs parois de côté, formant une partie du boîtier d'insonorisation et un ensemble de parois formant une partie du conduit de refroidissement du moteur, et dont l'autre inclut un ensemble constitué d'une paroi de fond et de plusieurs parois de côté, de préférence les deux parties de boîtier comprennent quatre parois de côté.
- les parties de boîtier sont aménagées par moulage.
- le conduit de refroidissement du moteur est formé en une seule et même pièce avec le boîtier d'insonorisation.
- le conduit de refroidissement du moteur présente une forme ajustée venant épouser approximativement la forme du moteur et de la volute du ventilateur tout en aménageant un espace autour du moteur et de la volute au sein duquel peut s'effectuer une circulation de gaz.
- les deux parties de logement forment conjointement, après assemblage des parties de boîtier, un logement propre à recevoir de manière ajustée, une partie correspondante du ventilateur avec interposition d'un un élément souple d'amortissement de vibrations.
- la paroi interne du conduit de refroidissement du moteur présente une forme venant épouser le contour externe du moteur et de la volute du ventilateur, tout en étant espacé de la surface externe dudit contour du moteur et de la volute de manière à former entre eux un passage pour le gaz, c'est-à-dire un espacement apte à permettre une circulation de gaz au contact de la surface externe du moteur et donc son refroidissement.
- les deux parties de boîtier sont solidarisées l'une à l'autre autour du ventilateur, en particulier par vissage, notamment au moyen de vis, boulots, écrous ou tout autre moyen de fixation similaire.

Les caractéristiques et le fonctionnement d'appareil de délivrance régulée d'un gaz selon l'invention seront mieux compris grâce à la description détaillée suivante faite en références aux Figures annexées parmi lesquelles :
- la figure 1 est une vue de l'appareil de l'invention en perspective éclatée ;
- la figure 2 est une vue d'un demi-boîtier de cet appareil, avant mise en place d'un ventilateur au sein de l'appareil ;
- la figure 3 est une vue du demi-boîtier similaire à la figure 2, après mise en place du ventilateur, et
- la figure 4 est une vue de l'appareil en coupe transversale passant par l'axe de rotation d'une roue à ailettes que comprend le ventilateur.

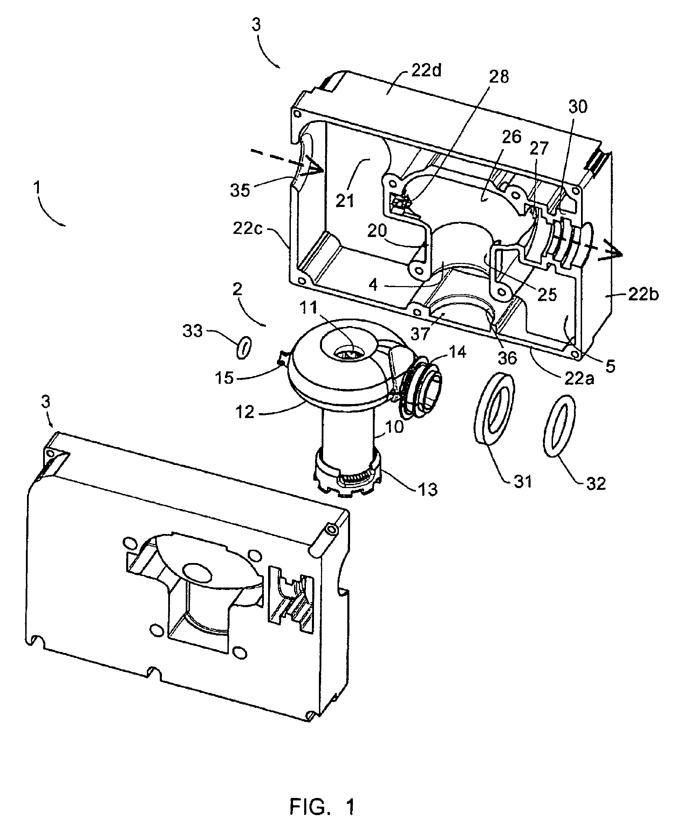

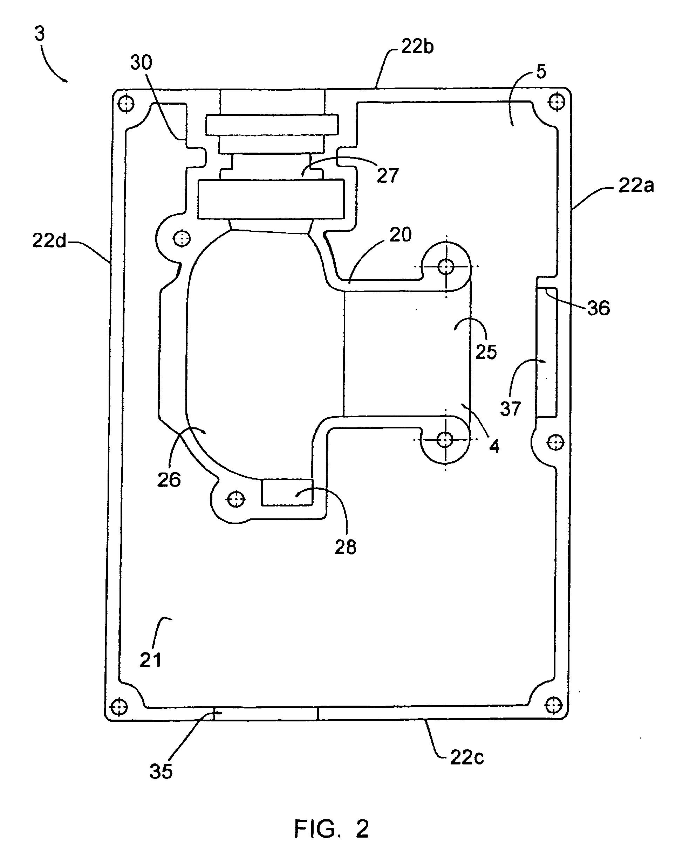

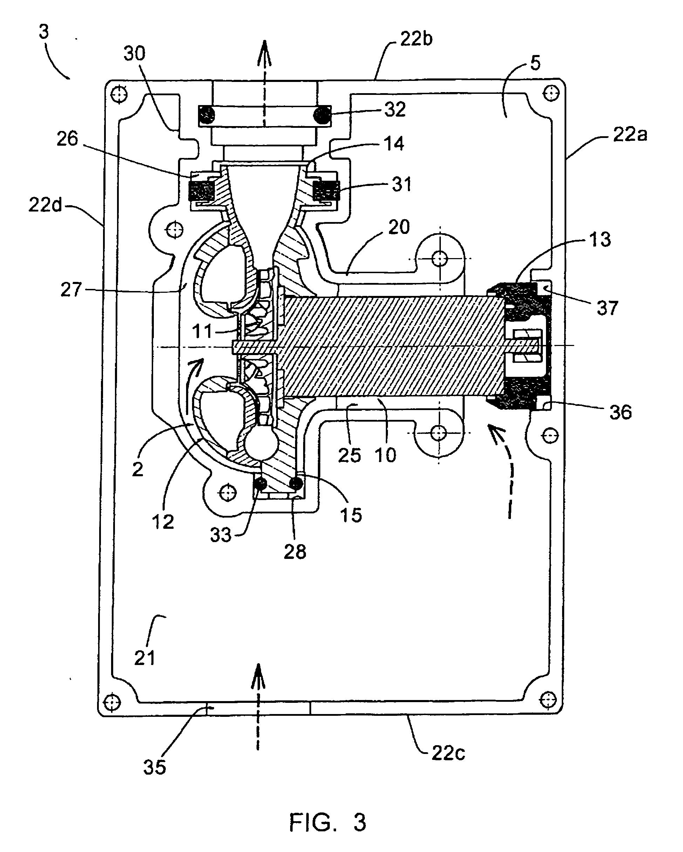

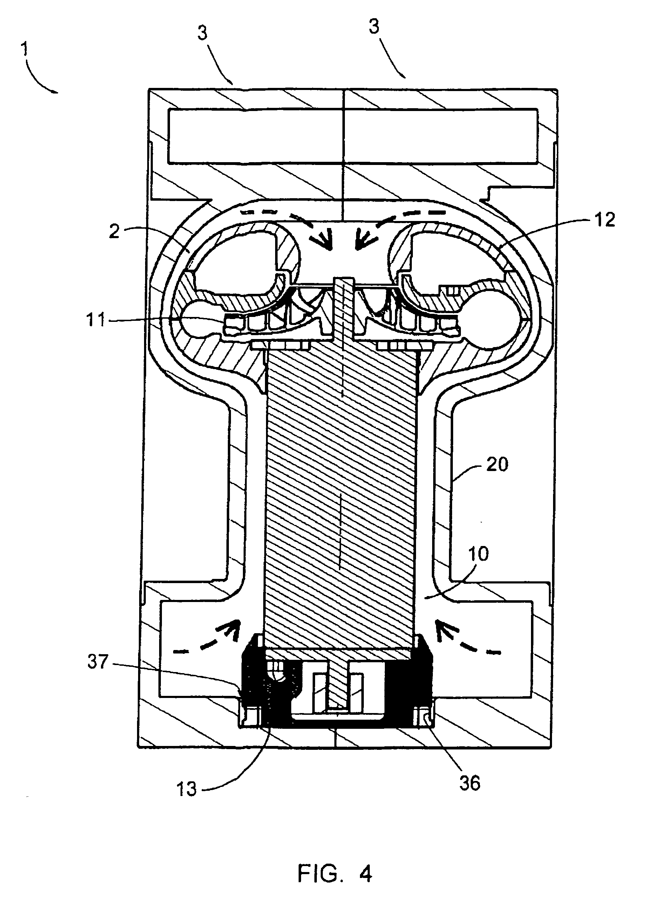

Les Figures 1 à 4 représentent un appareil 1 de délivrance régulée d'un gaz, notamment un appareil d'assistance respiratoire, comprenant un ventilateur 2 et deux demi-boîtiers 3 assemblables l'un à l'autre autour de ce ventilateur 2, l'assemblage de ces demi-boîtiers 3 permettant de former un conduit 4 de refroidissement du moteur du ventilateur 2, et un boîtier d'insonorisation 5.

Sur ces figures, les flèches en traits interrompus figurent l'écoulement du gaz, c'est à dire de l'air.

Le ventilateur 2 est constitué essentiellement par un moteur 10, une roue à ailettes 11 et une volute 12 contenant cette roue 11. Le moteur 10 présente une forme générale cylindrique et comprend un arbre axial sur lequel est montée la roue 11. À sa partie inférieure, il reçoit, emmanchée sur lui, une garniture 13 en élastomère. La roue à ailettes 11 a une entrée d'air axiale, disposée dans l'entrée de la volute 12, des conduits d'écoulement de l'air délimités par les ailettes et un flasque supérieur qu'elle comprend, et des sorties périphériques. La volute 12 comprend une entrée d'air axiale, un corps annulaire et une tubulure 14 de sortie d'air. L'ensemble des organes ci-dessus est bien connu en lui-même et ne sera donc pas décrit de manière plus détaillée.

Le ventilateur 2 comprend également un plot 15 de calage du ventilateur 2 par rapport au conduit 4, solidaire de la volute 12. Ce plot 15 présente une forme substantiellement cylindrique et s'étend radialement vers l'extérieur de la volute 12, en étant située en un emplacement diamétralement opposé à la tubulure 14.

Chaque demi-boîtier 3 est formé en une seule pièce par moulage d'une matière synthétique et comprend une série de trous de vis permettant son assemblage à l'autre demi-boîtier. Il comprend un ensemble de parois 20 formant une moitié du conduit de refroidissement 4, et un ensemble constitué d'une paroi de fond 21 et de quatre parois de côté 22a, 22b, 22c, 22d, formant une moitié du boîtier d'insonorisation 5.

L'ensemble de parois 20 présente une forme générale ajustée à la forme d'une moitié du moteur 10, de la volute 12, de la tubulure 14 et du plot 15. Il délimite ainsi :
- un logement amont 25 (par rapport au sens écoulement de l'air au travers de l'appareil 1), de forme substantiellement hémicylindrique, destiné à recevoir une large portion du corps du moteur 10,
- un logement médian 26 destiné à recevoir la volute 12,
- un logement aval 27 destiné à recevoir la tubulure 14, et
- un logement 28 destiné à recevoir le plot 15.

L'ouverture amont du logement 25 est située à distance de la paroi de côté 22a voisine ; elle comporte un pavillon d'entrée d'air afin de favoriser l'écoulement silencieux de l'air au travers d'elle.

Le logement 26 est de forme hémi-annulaire et est coaxial au logement 25.

Le logement 27 s'étend radialement par rapport au logement 26 et communique avec une paroi 30 formant une cavité hémi-tubulaire, cette cavité étant destinée à constituer, avec la partie 30 homologue de l'autre demi-boîtier 3, une ouverture de sortie de l'air hors du boîtier 5, débouchant dans la paroi de côté 22b. Les parois délimitant le logement 27 forment une cavité hémi-annulaire destinée à recevoir une garniture 31 en matériau souple, de montage de cette tubulure 14, d'étanchéité et d'amortissement des vibrations générées par le fonctionnement du ventilateur 2. La paroi 30 comprend également une telle cavité hémi-annulaire, pour la réception d'un joint d'étanchéité 32.

Le logement 28 est aménagé diamétralement à l'opposé du logement 27, coaxialement à celui-ci, et est adapté pour recevoir le plot 15 avec interposition d'une garniture 33 en matériau souple, de montage du ventilateur 2 et d'amortissement des vibrations générées par le fonctionnement de celui-ci.

La face d'assemblage que comprend chacun des deux demi-boîtiers 3 s'étend dans un plan passant sensiblement par l'axe des logements 25 à 28.

La paroi de côté 22c située à l'opposé de la paroi 22b par rapport au conduit 4 présente une encoche hémicirculaire 35 destinée à constituer, avec l'encoche homologue de l'autre demi-boîtier 3, une ouverture d'entrée de l'air dans le boîtier 5.

En outre, la paroi de côté 22a comprend une paroi hémicirculaire 36 coaxiale au logement 25, destiné à délimiter, avec la paroi hémicirculaire homologue de l'autre demi-boîtier 3, un logement circulaire 37 de réception ajustée de la garniture 13.

En pratique, le ventilateur 2 est mis en place dans l'un des demi-boîtiers 3 par engagement des garnitures 13, 31 et 33 dans des logements respectifs 37, 27 et 28, puis l'autre demi-boîtier 3 est assemblé et solidarisé au premier demi-boîtier 3 par la mise en place de vis ou analogues, pour former ainsi intégralement le conduit 4, le boîtier 5, les différents logements de montage du ventilateur 2 et les ouvertures d'entrée et de sortie d'air.

Le ventilateur 2 peut ainsi être monté de manière facile dans le conduit 4, et, une fois cet assemblage réalisé, ce ventilateur 2 est parfaitement maintenu en place dans ce conduit 4, avec amortissement des vibrations. Dans cette position de montage, les parois 20 s'étendent à proximité de la paroi du moteur 10 et de la volute 12, ménageant ainsi un passage d'écoulement forcé de l'air à proximité immédiate de ce moteur 10 et de cette volute 12, et donc assurant un refroidissement important de ceux-ci. Le fait que ce conduit 4 soit formé en une seule et même pièce avec le boîtier d'insonorisation 5 permet, par convection forcée, l'évacuation de la chaleur générée par le moteur 10.

La conception de l'appareil 1 sous forme de deux demi-boîtiers 3 assemblables a également pour avantages que le nombre de pièces constituant l'appareil est notablement réduit, qu'il est obtenu une plus grande facilité d'assemblage, et donc une durée d'assemblage réduite, et qu'une quantité moindre de matière est nécessaire pour constituer le conduit 4 de refroidissement du moteur 10 et le boîtier 5 d'insonorisation.

En outre, l'aménagement des demi-boîtiers 3 par moulage permet une possibilité augmentée de positionnement du conduit 4 dans le boîtier 5 et une possibilité d'intégrer à l'appareil 1 de façon optimale une entrée d'air ayant une forte atténuation acoustique et une perte de charge minimisée.

L'invention a été décrite ci-dessus en référence à une forme de réalisation fournie à titre d'exemple. Il va de soi qu'elle n'est pas limitée à cette forme de réalisation et qu'elle inclut toutes les variations et modifications couvertes par les revendications annexées. Notamment, les parties de boîtier peuvent ne pas être des "demi-boîtiers", c'est-à-dire que l'une de ces parties peut présenter une épaisseur différente de celle de l'autre partie ; les parties de boîtier peuvent être conformées de manière à être assemblables l'une à l'autre selon d'autres plans d'assemblage que celui représenté, par exemple l'appareil peut comprendre une partie de boîtier gauche et une partie de boîtier droit, assemblables selon un plan d'assemblage perpendiculaire à celui représenté sur les figures ; les parties de boîtier peuvent aussi être conformées de manière à être assemblables l'une à l'autre selon un plan perpendiculaire à l'axe des logements 25 et 26 du conduit 4, de sorte qu'il existerait ainsi une partie de boîtier supérieure et une partie de boîtier inférieure.

## Revendications

1. Appareil (1) de délivrance d'un gaz, notamment un appareil d'assistance respiratoire, comprenant un ventilateur (2) comprenant un moteur (10), une roue à ailettes (11) et une volute (12) contenant cette roue (11), un conduit (4) de refroidissement du moteur, et un boîtier d'insonorisation (5), contenant le ventilateur (2) et le conduit (4) de refroidissement du moteur, le conduit (4) de refroidissement du moteur présentant une forme ajustée à la forme du moteur (10) et de la volute (12) du ventilateur (2), de sorte que les parois (20) délimitant ce conduit (4) de refroidissement du moteur s'étendent à proximité de la paroi du moteur (10) et de la volute (12), ménageant ainsi un conduit d'écoulement autour de ce moteur (10) et de cette volute 12), **caractérisé en ce que** l'appareil (1) comprend deux parties de boîtier (3), dont l'une inclut un ensemble comprenant une paroi de fond (21) et au moins une paroi de côté (22a, 22b, 22c, 22d), formant une partie du boîtier d'insonorisation (5) et un ensemble de parois (20) formant une partie du conduit (4) de refroidissement du moteur, et dont l'autre inclut un ensemble comprenant une paroi de fond (21) et au moins une paroi de côté (22a, 22b, 22c, 22d), formant la partie complémentaire du boîtier d'insonorisation (5), et un ensemble de parois (20) formant la partie complémentaire du conduit (4) de refroidissement du moteur, lesdites parties du boîtier d'insonorisation (5) et du conduit (4) de refroidissement du moteur d'une même partie de boîtier (3) formant corps l'une avec l'autre, les deux parties de boîtier (3) étant assemblables l'une à l'autre autour du ventilateur (2) de manière à former conjointement ledit conduit (4) de refroidissement du moteur et ledit boîtier d'insonorisation (5).

2. Appareil selon la revendication 1, **caractérisé en ce que** les parties de boîtier sont des demi-boîtiers (3) assemblables l'un à l'autre selon un plan d'assemblage passant sensiblement par l'axe longitudinal du conduit (4) de refroidissement du moteur.

3. Appareil selon la revendication 1, **caractérisé en ce que** les parties de boîtier sont assemblables l'une à l'autre selon un plan d'assemblage sensiblement perpendiculaire à l'axe longitudinal du conduit de refroidissement du moteur.

4. Appareil selon l'une des revendications 1 à 3, **caractérisé en ce que** chaque partie de boîtier (3) forme au moins une partie de logement (25 à 28 ; 37), et **en ce que** les deux parties de logement (25 à 28 ; 37) forment conjointement, après assemblage des parties de boîtier (3), un logement propre à recevoir de manière ajustée, une partie correspondante du ventilateur (2), soit directement, soit avec interposition d'un élément de montage (13, 31, 33), notamment un élément souple d'amortissement de vibrations.

5. Appareil selon la revendication 4, **caractérisé en ce que** chaque partie de boîtier (3) comprend une partie de logement inférieur, et **en ce que** les deux parties de logement inférieur forment conjointement, après assemblage des parties de boîtier (3), un logement (37) apte à recevoir de manière ajustée l'extrémité du moteur (10) opposée à la volute (12) ou une pièce (13) fixée à cette extrémité.

6. Appareil selon la revendication 4 ou la revendication 5, **caractérisé en ce que** chaque partie de boîtier (3) comprend une partie de logement de tubulure (14) de sortie de volute (12), les deux parties de logement de tubulure (14) de sortie de volute (12) formant conjointement, après assemblage des parties de boîtier, un logement (27) apte à recevoir de manière ajustée la tubulure (14) de sortie de la volute (12), avec interposition d'une garniture (31) de montage et d'amortissement.

7. Appareil selon l'une des revendications 4 à 6, **caractérisé en ce que** chaque partie de boîtier (3) comprend une partie de logement de calage de ventilateur (2), les deux parties de logement de calage de ventilateur (2) formant conjointement, après assemblage des parties de boîtier, un logement (28) apte à recevoir de manière ajustée un plot (15) de calage de ventilateur (2) solidaire de la volute (12), avec interposition d'une garniture (33) de montage et d'amortissement.

8. Appareil selon la revendication 7, **caractérisé en ce que** chaque partie de logement de calage de ventilateur (2) est aménagée en un emplacement du conduit (4) de refroidissement du moteur diamétralement opposé à la partie de logement de tubulure (14) de sortie de volute (12).

9. Appareil selon la revendication 1, **caractérisé en ce que** l'appareil (1) comprend deux parties de boîtier (3), dont l'une inclut un ensemble comprenant une paroi de fond (21) et plusieurs parois de côté (22a, 22b, 22c, 22d), formant une partie du boîtier d'insonorisation (5) et un ensemble de parois (20) formant une partie du conduit (4) de refroidissement du moteur, et dont l'autre inclut un ensemble comprenant d'une paroi de fond (21) et de plusieurs parois de côté (22a, 22b, 22c, 22d).

10. Appareil selon la revendication 9, **caractérisé en ce que** les deux parties de boîtier (3) comprennent 4 parois de côté (22a, 22b, 22c, 22d).

11. Appareil selon la revendication 1, **caractérisé en ce que** les parties de boîtier sont aménagées par moulage.

12. Appareil selon la revendication 1, **caractérisé en ce que** le conduit de refroidissement du moteur est formé en une seule et même pièce avec le boîtier d'insonorisation.

13. Appareil selon la revendication 4, **caractérisé en ce que** les deux parties de logement (25 à 28 ; 37) forment conjointement, après assemblage des parties de boîtier (3), un logement propre à recevoir de manière ajustée, une partie correspondante du ventilateur (2) avec interposition d'un un élément souple d'amortissement de vibrations (13, 31, 33).

14. Appareil selon la revendication 1, **caractérisé en ce que** la paroi interne du conduit (4) de refroidissement du moteur présente une forme venant épouser le contour externe du moteur (10) et de la volute (12) du ventilateur (2), tout en étant espacé de la surface externe dudit contour du moteur (10) et de la volute (12) de manière à former entre eux un passage pour le gaz.

15. Appareil selon la revendication 1, **caractérisé en ce que** les deux parties de boîtier (3) sont solidarisées l'une à l'autre autour du ventilateur (2).

## Patentansprüche

1. Gerät (1) zur Lieferung eines Gases, insbesondere Gerät zur Atmungsunterstützung, umfassend einen Ventilator (2), umfassend einen Motor (10), ein Flügelrad (11) und ein Spirale (12), die dieses Rad (11) enthält, eine Leitung (4) zum Kühlen des Motors und ein Schalldämpfergehäuse (5), das den Ventilator (2) und die Motorkühlleitung enthält, wobei die Motorkühlleitung (4) eine an die Form des Motors (10) und der Spirale (12) des Ventilators (2) angepasste Form hat, so dass sich die Wände (20), die diese Motorkühlleitung (4) begrenzen, in der Nähe der Wand des Motors (10) und der Spirale (12) erstrecken, wobei so eine Abflussleitung um diesen Motor (10) und diese Spirale (12) ausgenommen wird, **dadurch gekennzeichnet, dass** das Gerät (1) zwei Gehäuseteile (3) umfasst, von denen der eine Einheit, umfassend eine Bodenwand (21) und mindestens eine Seitenwand (22a, 22b, 22c, 22d), die einen Teil des Schalldämpfergehäuses (5) bildet, und eine Gesamtheit von Wänden (20), die einen Teil der Motorkühlleitung (4) bilden, einschließt, wobei der andere eine Einheit, umfassend eine Bodenwand (21) und mindestens eine Seitenwand (22a, 22b, 22c, 22d), die den komplementären Teil des Schalldämpfergehäuses (5) bildet, und eine Gesamtheit von Wänden (20), die den komplementären Teil der Motorkühlleitung (4) bilden, einschließt, wobei die Teile des Schalldämpfergehäuses (5) und der Motorkühlleitung (4) eines selben Gehäuseteils (3) einen Körper miteinander bilden, wobei die beiden Gehäuseteile (3) miteinander um den Ventilator (2) zusammengefügt sind, um gemeinsam die Motorkühlleitung (4) und das Schalldämpfergehäuse (5) zu bilden.

2. Gerät nach Anspruch 1, **dadurch gekennzeichnet, dass** die Gehäuseteile halbe Gehäuse (3) sind, die miteinander entlang einer Montageebene, die im Wesentlichen durch die Längsachse der Motorkühlleitung (4) verläuft, zusammengebaut werden können.

3. Gerät nach Anspruch 1, **dadurch gekennzeichnet, dass** die Gehäuseteile miteinander entlang einer Montageebene im Wesentlichen senkrecht auf die Längsachse der Motorkühlleitung zusammengebaut werden können.

4. Gerät nach einem der Ansprüche 1 bis 3, **dadurch gekennzeichnet, dass** jeder Gehäuseteil (3) mindestens einen Lagerungsteil (25 bis 28; 37) bildet, und dass die beiden Lagerungsteile (25 bis 28; 37) gemeinsam nach dem Zusammenbau der Gehäuseteile (3) eine Lagerung bilden, die geeignet ist, auf angepasste Weise einen entsprechenden Teil des Ventilators (2) entweder direkt oder unter Zwischenschaltung eines Montageelements (13, 31, 33), insbesondere eines flexiblen Schwindungsdämpfelements, aufzunehmen.

5. Gerät nach Anspruch 4, **dadurch gekennzeichnet, dass** jeder Gehäuseteil (3) einen unteren Lagerungsteil umfasst, und dass die beiden unteren Lagerungsteile gemeinsam nach dem Zusammenbau der Gehäuseteile (3) eine Lagerung (37) bilden, die geeignet ist, auf angepasste Weise das Ende des Motors (10) gegenüber der Spirale (12) oder einen an diesem Ende befestigten Teil (13) aufzunehmen.

6. Gerät nach Anspruch 4 oder Anspruch 5, **dadurch gekennzeichnet, dass** jeder Gehäuseteil (3) Lagerungsteil eines Ausgangsrohrs (14) der Spirale (12) umfasst, wobei die beiden Ausgangsrohrlagerungsteile (14) der Spirale (12) gemeinsam nach dem Zusammenbau der Gehäuseteile eine Lagerung (27) bilden, die geeignet ist, auf angepasste Weise das Ausgangsrohr (14) der Spirale (12) unter Zwischenschaltung einer Dichtung (31) zur Montage und Dämpfung aufzunehmen.

7. Gerät nach einem der Ansprüche 4 bis 6, **dadurch gekennzeichnet, dass** jeder Gehäuseteil (3) einen Lagerungsteil einer Ventilatorbefestigung (2) umfasst, wobei die beiden Lagerungsteile einer Ventilatorbefestigung (2) gemeinsam nach dem Zusammenbau der Gehäuseteile eine Lagerung (28) bilden, die geeignet ist, auf angepasste Weise einen Befestigungsklotz (15) des Ventilators (2), der mit der Spirale (12) verbunden ist, unter Zwischenschaltung einer Dichtung (33) zur Montage und Dämpfung aufzunehmen.

8. Gerät nach Anspruch 7, **dadurch gekennzeichnet, dass** jeder Lagerungsteil einer Ventilatorbefestigung (2) an einer Stelle der Motorkühlleitung (4) diametral gegenüber dem Lagerungsteil einer Ausgangsleitung (14) der Spirale (12) angeordnet ist.

9. Gerät nach Anspruch 1, **dadurch gekennzeichnet, dass** das Gerät (1) zwei Gehäuseteile (3) umfasst, von denen einer eine Einheit, umfassend eine Bodenwand (21) und mehrere Seitenwände (22a, 22b, 22c, 22d), die einen Teil des Schalldämpfergehäuses (5) bilden, und eine Gesamtheit von Wänden (20), die einen Teil der Motorkühlleitung (4) bilden, einschließt, und von denen der andere eine Einheit, umfassend eine Bodenwand (21) und mehrere Seitenwände (22a, 22b, 22c, 22d), einschließt.

10. Gerät nach Anspruch 9, **dadurch gekennzeichnet, dass** die beiden Gehäuseteile (3) 4 Seitenwände (22a, 22b, 22c, 22d) umfassen.

11. Gerät nach Anspruch 1, **dadurch gekennzeichnet, dass** die Gehäuseteile durch Formguss hergestellt werden.

12. Gerät nach Anspruch 1, **dadurch gekennzeichnet, dass** die Motorkühlleitung aus einem einzigen und selben Stück mit dem Schalldämpfergehäuse gebildet ist.

13. Gerät nach Anspruch 4, **dadurch gekennzeichnet, dass** die beiden Lagerungsteile (25 bis 28; 37) gemeinsam nach dem Zusammenbau der Gehäuseteile (3) eine Lagerung bilden, die geeignet ist, auf angepasste Weise einen entsprechenden Teil des Ventilators (2) unter Zwischenschaltung eines flexiblen Schwingungsdämpfelements (13, 31, 33) aufzunehmen.

14. Gerät nach Anspruch 1, **dadurch gekennzeichnet, dass** die Innenwand der Motorkühlleitung (4) eine Form aufweist, die sich an die äußere Kontor des Motors (10) und der Spirale (12) des Ventilators (2) anlegt, wobei sie zu der äußeren Fläche der Kontur des Motors (10) und der Spirale (12) beabstandet ist, um zwischen ihnen einen Durchgang für das Gas zu bilden.

15. Gerät nach Anspruch 1, **dadurch gekennzeichnet, dass** die beiden Gehäuseteile (3) miteinander um den Ventilator (2) herum verbunden sind.

## Claims

1. Apparatus (1) for delivering a gas, in particular an assisted breathing apparatus, comprising a fan (2) comprising a motor (10), a vaned rotor (11) and a volute (12) containing this rotor (11), a duct (4) for cooling the motor, and a soundproofing housing (5) containing the fan (2) and the motor-cooling duct (4), the motor-cooling duct (4) having a shape matched to the shape of the motor (10) and of the volute (12) of the fan (2), such that the walls (20) defining this motor-cooling duct (4) extend close to the wall of the motor (10) and of the volute (12), thus providing a flow duct around this motor (10) and this volute (12), **characterised in that** the apparatus (1) comprises two casing portions (3), one of which includes a set comprising a back wall (21) and at least one side wall (22a, 22b, 22c, 22d), forming a portion of the soundproofing housing (5), and a set of walls (20) forming a portion of the motor-cooling duct (4), and the other of which includes a set comprising a back wall (21) and at least one side wall (22a, 22b, 22c, 22d), forming the complementary portion of the soundproofing housing (5), and a set of walls (20) forming the complementary portion of the motor-cooling duct (4), said portions of the soundproofing housing (5) and of the motor-cooling duct (4) of a same casing portion (3) being integral with one another, the two casing portions (3) being able to be assembled together around the fan (2) so as jointly to form said motor-cooling duct (4) and said soundproofing housing (5).

2. Apparatus according to claim 1, **characterised in that** the casing portions are half-casings (3) that can be assembled together in an assembly plane passing substantially through the longitudinal axis of the motor-cooling duct (4).

3. Apparatus according to claim 1, **characterised in that** the casing portions can be assembled together in an assembly plane substantially perpendicular to the longitudinal axis of the motor-cooling duct.

4. Apparatus according to any of claims 1 to 3, **characterised in that** each casing portion (3) forms at least one housing portion (25 to 28; 37) and **in that** the two housing portions (25 to 28; 37) jointly form, after assembly of the casing portions (3), a housing suitable for closely accommodating a corresponding portion of the fan (2), either directly or with interposition of a mounting element (13, 31, 33), in particular a flexible vibration-damping element.

5. Apparatus according to claim 4, **characterised in that** each casing portion (3) comprises a lower housing portion and **in that** the two lower housing portions jointly form, after assembly of the casing portions (3), a housing (37) capable of closely accommodating the opposite end of the motor (10) from the volute (12) or a piece (13) fixed to this end.

6. Apparatus according to either claim 4 or claim 5, **characterised in that** each casing portion (3) comprises a portion for housing an outlet nozzle (14) of the volute (12), the two portions for housing the outlet nozzle (14) of the volute (12) jointly forming, after assembly of the casing portions, a housing (27) capable of closely accommodating the outlet nozzle (14) of the volute (12), with interposition of a mounting/damping seal (31).

7. Apparatus according to any of claims 4 to 6, **characterised in that** each casing portion (3) comprises a housing portion for fitting the fan (2), the two housing portions for fitting the fan (2) jointly forming, after assembly of the casing portions, a housing (28) capable of closely accommodating a stud (15) for fitting the fan (2), rigidly connected to the volute (12), with interposition of a mounting/damping seal (33).

8. Apparatus according to claim 7, **characterised in that** each housing portion for fitting the fan (2) is provided at a location in the motor-cooling duct (4) which is diametrically opposed to the portion for housing the outlet nozzle (14) of the volute (12).

9. Apparatus according to claim 1, **characterised in that** the apparatus (1) comprises two casing portions (3), one of which includes a set comprising a back wall (21) and a plurality of side walls (22a, 22b, 22c, 22d), forming a portion of the soundproofing housing (5), and a set of walls (20) forming a portion of the motor-cooling duct (4), and the other of which includes a set comprising a back wall (21) and a plurality of side walls (22a, 22b, 22c, 22d).

10. Apparatus according to claim 9, **characterised in that** the two casing portions (3) comprise four side walls (22a, 22b, 22c, 22d).

11. Apparatus according to claim 1, **characterised in that** the casing portions are provided by moulding.

12. Apparatus according to claim 1, **characterised in that** the motor-cooling duct is formed in one and the same piece with the soundproofing housing.

13. Apparatus according to claim 4, **characterised in that** the two housing portions (25 to 28; 37) jointly form, after assembly of the casing portions (3), a housing suitable for closely accommodating a corresponding portion of the fan (2), with interposition of a flexible vibration-damping element (13, 31, 33).

14. Apparatus according to claim 1, **characterised in that** the internal wall of the motor-cooling duct (4) has a shape that matches the external contour of the motor (10) and of the volute (12) of the fan (2), while being spaced apart from the external surface of said contour of the motor (10) and of the volute (12) so as to form between them a passage for the gas.

15. Apparatus according to claim 1, **characterised in that** the two casing portions (3) are rigidly interconnected around the fan (2).
